# EUROPEAN PATENT SPECIFICATION

(11) **EP 2 363 113 B1**
(45) Date of publication and mention of the grant of the patent: **02.08.2017**
(21) Application number: 11165824.1
(22) Date of filing: 04.12.2007
(51) Int. Cl.: A61K 9/00, A61K 9/48, A61K 31/7048

(54) **Enhanced immediate release formulations of topiramate**
Topiramat-Formulierungen für verstärkte unmittelbare Freisetzung
Formulations à libération immédiate de topiramate améliorées

(30) Priority: 04.12.2006 US 872497 P
(43) Date of publication of application: 07.09.2011
(62) Divisional of application: 07868979.1
(73) Proprietor: Supernus Pharmaceuticals, Inc., Rockville, MD 20850 (US)
(72) Inventor: Liang, Likan, Boyds, MD 20841 (US); Bhatt, Padmanabh, Rockville, MD 20850 (US); Wang, Hua, Clarksville, MD 21029 (US)
(74) Representative: Louis Pöhlau Lohrentz

(56) References cited:
- WO-A-02/03984
- WO-A-02/43731
- WO-A-2006/009403
- US-A1- 2005 191 343
- US-A1- 2006 233 892

## Description

### BACKGROUND OF THE INVENTION

Topiramate is a sulfamate substituted monosaccharide which under the tradename TOPAMAX® (Ortho-McNeil Pharmaceutical, Inc., Raritan, NJ, U.S.A.) has been approved for use as an antiepileptic agent, as an adjuvant therapy for patients with partial onset seizures or primary generalized tonic-clonic seizures, and for the prevention of migraine. See generally, Physician's Desk Reference, 60th ed., 2538-2447 (2006); see also, U.S. Pat. No. 4,513,006.

For the treatment of epilepsy, the recommended dose of Topamax® is 400 mg/day in one or more doses (Physician's Desk Reference, 60th ed., 2538-2447 (2006)). For the treatment of epilepsy in adults, treatment is initiated with a dose of 25-50 mg/day, with the dose titrated in increments of 25-50 mg at weekly intervals to the recommended or effective dose. Topamax® is an immediate release formulation. Adverse effects associated with the administration of Topamax® include, but are not limited to, somnolence, dizziness, ataxia, speech disorders and related speech problems, psychomotor slowing, abnormal vision, difficulty with memory, paresthesia, diplopia, renal calculi (kidney stones), hepatic failure, pancreatitis, renal tubular acidosis, acute myopia and secondary angle closure glaucoma (Physician's Desk Reference, 10th ed., 2538-2447 (2006)).

Topiramate is a white crystalline powder that is soluble in alkaline solutions containing sodium hydroxide or sodium phosphate, soluble in acetone, dimethylsulfoxide and ethanol. However, the solubility of topiramate in water at room temperature is only about 9.8 mg/ml.

Topiramate has been investigated for use as an anti-obesity agent, a blood pressure lowering agent, and a mood stabilizer, including use as an antimanic, antidepressant, and for the treatment of post-traumatic stress disorder, migraines (Rev Neurol. 2006 Aug 16-31;43(4):193-6), cluster headaches, and neuropathic pain. See, e.g., U.S. PatNos. 6,191,117; 6,201,010; 5,753,693; 5,998,380; 6,319,903; 5,935,933; and 5,760,007. However, the time it takes for topiramate to reach peak plasma levels (i.e., about two hours) may be too slow for its effective use in the treatment of some conditions, such as neuropathic pain or migraine. Moreover, the compound's relatively low aqueous solubility makes it difficult to provide a dosage form, which may be necessary for the effective treatment of many conditions, and which may allow a reduction in adverse effects associated with peak plasma levels of the drug. Therefore, new highly soluble and bioavailable forms of topiramate are needed in order to increase the safety and effectiveness of the compound.

### SUMMARY OF THE INVENTION

In one embodiment, the current invention provides a novel enhanced immediate release formulation of topiramate for an oral administration to a mammalian subject, wherein at least 80% of the active compound is dissolved in a time period of not more than 30 minutes. The formulation comprises topiramate as an active ingredient and at least one agent selected from complexing agents, enhancing agents and combinations thereof.

In one embodiment of the invention, the formulation comprises topiramate and at least one complexing agent.

In another embodiment of the invention, the formulation comprises topiramate and at least one enhancing agent selected from the group consisting of solubility enhancing agents, dissolution enhancing agents, absorption enhancing agents, penetration enhancing agents, surface active agents, stabilizing agents, enzyme inhibitors, p-glycoprotein inhibitors, multidrug resistance protein inhibitors and combinations thereof.

In yet another embodiment, the invention provides an enhanced immediate release formulation that comprises topiramate as an active ingredient, at least one complexing agent and at least one enhancing agent.

It is an additional object of the present invention to provide a dosage form containing an enhanced immediate release formulation of topiramate. In one embodiment, said dosage form is an oral dosage form that may be selected from a tablet, a pill, a capsule, a caplet, a troche, a sachet, a cachet, a pouch, a powder, a bead, a solution, a gum, sprinkles and an orally disintegrating dosage form.

It is also an object of the instant invention to provide a method of treatment of a pathological condition in a mammalian subject by administering to said subject an enhanced immediate release topiramate formulation. In one embodiment, this condition is an acute condition. In a further embodiment, said condition is a migraine.

### BRIEF DESCRIPTION OF THE DRAWINGS

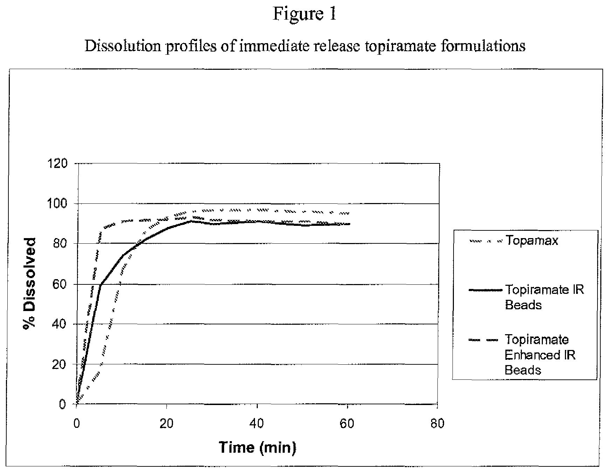

Figure 1 shows the dissolution profiles of Topamax®, topiramate IR beads, and topiramate enhanced immediate release beads.

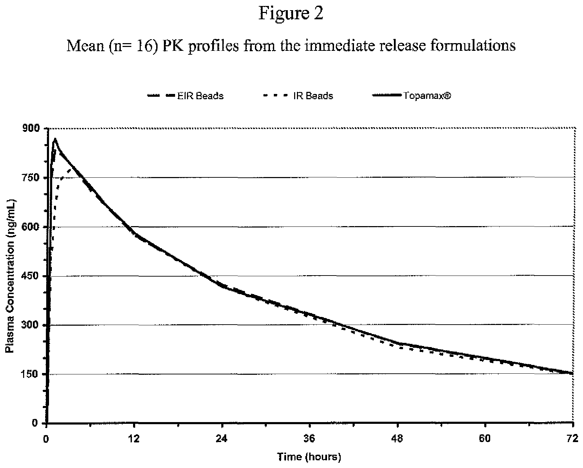

Figure 2 shows mean (n= 16) pharmacokinetic profiles for the immediate release formulations.
Figure 3 shows the dissolution profiles of the cyclodextrin-containing enhanced formulations of topiramate.
Figure 4 shows the dissolution profiles of the non-complexed enhanced formulations of topiramate.

### DETAILED DESCRIPTION OF THE INVENTION

For the purposes of this application, the term "topiramate" includes topiramate or any pharmaceutically acceptable salts or derivatives thereof, and the term "cyclodextrins" includes cyclodextrin derivatives.

An "immediate release formulation" refers to a formulation that releases greater than or equal to about 80% of the pharmaceutical agent in less than or equal to about 1 hour.

For the purposes of this application, an "enhancing agent" (an enhancer), is defined as any non-pharmaceutically active ingredient that improves the efficacy and therapeutic potential of a formulation.

The term "enhanced immediate release formulation" (EIR) as used herein describes an immediate release formulation improved in terms of efficacy and therapeutic potential.

As used herein, unless otherwise noted, "rate of release" or "release rate" of a drug refers to the quantity of drug released from a dosage form per unit time, e.g., milligrams of drug released per hour (mg/hr) or a percentage of a total drug dose released per hour. Drug release rates for dosage forms are typically measured as an *in vitro* rate of drug release, i.e., a quantity of drug released from the dosage form per unit time measured under appropriate conditions and in a suitable fluid. The time at which a specified percentage of the drug within a dosage form has been released from said dosage form is referred to as the "Tx" value, where "x" is the percent of drug that has been released.

The release rates referred to herein are determined by placing a dosage form to be tested in a medium in an appropriate dissolution bath. Aliquots of the medium, collected at pre-set intervals, are then injected into a chromatographic system fitted with an appropriate detector to quantify the amounts of drug released during the testing intervals.

"C" denotes the concentration of drug in blood plasma, or serum, of a subject, and is generally expressed as mass per unit volume, typically nanograms per milliliter. For convenience, this concentration may be referred to herein as "drug plasma concentration", "plasma drug concentration" or "plasma concentration" which is intended to be inclusive of a drug concentration measured in any appropriate body fluid or tissue. The plasma drug concentration at any time following drug administration is referenced as Cₜᵢₘₑ, as in C₉ₕᵣ or C₄ₕᵣ, etc.

The maximum plasma drug concentration during the dosing period is referenced as Cₘₐₓ, while Cₘᵢₙ refers to the minimum blood plasma drug concentration at the end of a dosing interval; and Cₐᵥₑ refers to an average concentration during the dosing interval.

Persons of skill in the art will appreciate that blood plasma drug concentrations obtained in individual subjects will vary due to interpatient variability in the many parameters affecting drug absorption, distribution, metabolism and excretion. For this reason, unless otherwise indicated, when a drug plasma concentration is listed, the value listed is the calculated mean value based on values obtained from a group of subjects tested.

The term "bioavailability" refers to an extent to which--and sometimes rate at which--the active species (drug or metabolite) enters systemic circulation, thereby gaining access to the site of action.

Side effect is defined herein as a secondary and usually adverse effect of a drug.

The term "beads", as used herein, includes, without any limitations on the nature, mode of the drug distribution, and size thereof, any particles, spheres, beads, granules, pellets, particulates or any structural units that may be incorporated into an oral dosage form.

The term "capsule" as used herein refers to the pharmaceutically inactive enclosure intended to contain at least one pharmaceutically active ingredient.

The instant invention provides an enhanced immediate release formulation of topiramate for oral administration to a mammalian subject, wherein at least 80% of an active compound is dissolved in a time period of not more than 30 minutes. Preferably, at least 50% of the active compound is released in a time period of not more than 10 minutes, and at least 25% is dissolved in a time period of not more than 5 minutes after the oral administration. In the most preferred embodiment of the present invention, at least 30% of the active compound is released in a time period of not more than 5 minutes. The formulation comprises topiramate as an active ingredient and at least one agent selected from complexing agents, enhancing agents and combinations thereof.

In one embodiment of the invention, the formulation comprises topiramate and at least one complexing agent. The complexing agent, without any limitations hereon, may be selected from benzoates, hydroxybenzoates, amines, amides or polyamines, such as polyvinylpyrrolidones, pyridoxine hydrochloride, nicotinamide, polyamines, e.g. polyvinyl amines and polyallylamines, polyethylene imines, polyvinyl pyridine, and polylysine, oligo- and polysaccharides such as cyclodextrins and their derivatives, aminopolysaccharides such as chitosan, polyanions such as NAFION®, oxa- and thia-crown ethers, polyoxoalkylenes, or polysiloxanes.

In an exemplary embodiment, the invention comprises a highly soluble complex of topiramate with a cyclodextrin which is selected from a group consisting of hydroxypropyl-beta-cyclodextrin, beta-cyclodextrin, gamma-cyclodextrin, and alpha-cyclodextrin, or its derivative. Cyclodextrins have been used for drug solubility improvement due to their strong tendency to interact and form inclusion complexes with the drugs (US 4727064).

The ratio of cyclodextrin to topiramate in the novel enhanced immediate release formulation is preferably less than 20:1, and more preferably less than 5:1.

The preferred complexing agent for this embodiment is hydroxypropyl-beta-cyclodextrin (HPBCD). HPBCD has a "closed" circular molecular structure. The glycosidic oxygen forming the bond between the adjacent glucose monomers and the hydrogen atoms lining the cavity of the cyclodextrin imparts an electron density and hydrophobic character to the cavity. Organic compounds interact with the walls of the cavity to form inclusion complexes. The hydroxyl groups and the hydroxypropyl groups are on the exterior of the molecule and interact with water to provide increased aqueous solubility of the complexes made with HPBCD.

The methods of preparation of highly soluble complexes of pharmaceutically active agents, including topiramate, with cyclodextrins are described in the art and typically involve either mixing or kneading the drug and the cyclodextrin together in the presence of water and/or an organic solvent, or adding an excess of the pharmaceutical agent to the solution of the cyclodextrin (e.g. US Patent numbers 4727064 and 5707975, and USPTO Publication No. 20060105045). Thus, the highly soluble complex of topiramate and cyclodextrin may be prepared by mixing topiramate and cyclodextrin together in the presence of water and, optionally, an organic solvent. The concentration of cyclodextrin is preferably high to facilitate the formation of topiramate-enhancer complex. In the case when the complexing agent is hydroxypropyl-beta-cyclodextrin, topiramate and HPBCD are mixed in such a way that during the complexation step the concentration of HPBCD is greater than 2%, preferably greater than 20%, and more preferably greater than at least about 40%, while the amount of topiramate is determined by a desired ratio of HPBCD to topiramate. For partial complexation of topiramate, the ratio by weight is preferably less than 20:1, and more preferably less than 5:1. The ratio has to be higher for the complete complexation of topiramate. The mixing time of the complex solution is from about one hour to about 48 hours, and preferably about 5 hours to about 24 hours.

An alternative method of preparation comprises an incremental addition of topiramate and cyclodextrin to a saturated dispersion of topiramate. The initial amount of hydroxypropyl-beta-cyclodextrin powder is added to the saturated topiramate dispersion. Addition of the HPBCD results in the reduction of the viscosity of the dispersion. Once the dispersion becomes significantly less viscous, more topiramate is added followed by sprinkling of more hydroxypropyl-beta-cyclodextrin. The drug and hydroxypropyl-beta-cyclodextrin addition steps are repeated, and the solution/dispersion is mixed for 12-18 hours. The solution can be dried by the methods described in the current invention at any point during this process thus producing a formulation with a pre-determined ratio of complexed/uncomplexed topiramate. It should be noted that, due to the nature of the process, the undissolved topiramate will be in the form of very fine particles of the size of, depending on the stage of the process, less than 50 microns, less than 10 microns, or even less than 5 microns. This reduced size of the undissolved particles provides for the larger surface area and thus enhanced dissolution, in addition to the dissolution and solubility enhancement caused by the formation of the complex. If complete complexation of topiramate is desired, the saturated topiramate-HPBCD complex solution is separated from the undissolved topiramate by an appropriate separation method, such as filtration and centrifugation, and on subsequent drying provides completely complexed topiramate. For the purposes of this invention, however, formulations with the HPBCD/ topiramate weight ratio of 3:2 is preferred.

In another embodiment of the invention, the novel formulation comprises topiramate and at least one enhancing agent selected from a group consisting of solubility enhancing agents, dissolution enhancing agents, absorption enhancing agents, penetration enhancing agents, surface active agents, stabilizing agents, enzyme inhibitors, p-glycoprotein inhibitors, multidrug resistance protein inhibitors and combinations thereof. The representative, but non-limiting examples of these compounds are Vitamin E TPGS, amino acids such as glutamic acid and glycine, sorbitol, mannose, amylose, maltose, mannitol, lactose, sucrose, glucose, xylitose, dextrins such as maltodextrin, Cremophor RH40 (glycerol-polyethylene glycol oxystearate), Gelucire 50/13 (PEG-32 glyceryl palmitostearate), sodium lauryl sulfate, Tween 80 (polyoxyethylene sorbitan monooleate), benzyl alcohol, Span 20 (sorbitan monolaurate), Poloxamer 407, polyethylene glycols, such as PEG3350; polyvinylpyrrolidones such as PVP K25, polyvinylalcohols, polyalcohols, oleic acid, Capmul GMO (glyceryl monooleate), sodium benzoate, cetyl alcohol, sucrose stearate, crospovidone, sodium starch glycolate, crosscarmellose sodium, carboxymethylcellulose, starch, pregelatinized starch, HPMC, substituted hydroxypropylcellulose, microcrystalline cellulose, sodium bicarbonate, calcium citrate, sodium docusate, and menthol, among others. Enhancers can be combined to achieve multiple enhancement effects, for example, solubility enhancement combined with permeability enhancement and p-glycoprotein inhibition, or to provide a synergistic effect to achieve greater and more efficient enhancement. For example, polyglycolized glycerides (different grades of Gelucire) can be combined with sodium lauryl sulfate to achieve higher solubility enhancement as well as faster dissolution of topiramate.

In yet another embodiment, the invention provides an enhanced immediate release formulation that comprises topiramate as an active ingredient, at least one complexing agent and at least one enhancing agent. The degree of complexation of topiramate in the formulation may vary considerably from 0.1% to up to 100%.

Comparative data for the topiramate solubility enhancement by complexing and enhancing agents are represented in Table 1.

**Table 1. Topiramate Solubility Enhancement**

| Enhancing/Complexing agent | Enhancement Ratio |
|---|---|
| 4% HPBCD | 1.72 |
| 2% HPBCD | 1.54 |
| 4% Vitamin E TPGS | 1.51 |
| 4% HPBCD + 0.48% Meglumine | 1.46 |
| 2% Vitamin E TPGS | 1.41 |
| 2% Cremophor RH40 | 1.33 |
| 2% Gelucire 50/13 | 1.3 |
| 0.5% SLS | 1.3 |
| 2% Vitamin E TPGS + 0.2% SLS | 1.28 |
| 1% Tween 80 | 1.26 |
| 1% Gelucire 50/13 | 1.22 |
| 0.15% Benzyl alcohol | 1.17 |
| 2% Cremophor RH + 0.2% Span 20 | 1.16 |
| 1% Poloxamer 407 | 1.16 |
| 1% PEG 3350 | 1.16 |
| 0.6% PVP K25 | 1.16 |
| 0.5% Oleic acid | 1.15 |
| 1% SLS | 1.14 |
| 1% Capmul GMO + 0.2% SLS | 1.14 |
| 0.6% Sodium benzoate | 1.14 |
| 0.6% Cetyl alcohol | 1.14 |
| 0.5% Sucrose stearate | 1.14 |
| 1% Gelucire 50/13 | 1.12 |
| 1% Capmul MCM + 0.2% SLS | 1.11 |
| 0.2% HPMC | 1.08 |
| 2% NaHCO3 | 1.06 |
| 0.24% Docusate sodium | 1.06 |
| 2% PEG 3350 | 1.05 |
| 0.24% Menthol | 1.04 |
| Control | 1 |

In a further embodiment of the invention, at least part (i.e. more than 0.01%, preferably, at least several percent) of the active ingredient may be present in the formulation in a form of micronized particles with the size of from 1 µm to 1000 µm, preferably from 2 µm to about 200 µm, more preferably from 2 µm to about 100 µm. For example, the formulation may be prepared in such a way that the majority of the micronized particles are less than 50 µm size, or less than 10 µm size, or less than 5 µm size. Further, one or more of the complexing agents, enhancers or combinations thereof may be present in the formulations covered by this embodiment. Said enhancers are selected from solubility enhancing agents, dissolution enhancing agents, absorption enhancing agents, penetration enhancing agents, surface active agents, such as non-ionic surfactants, ionic surfactants or combinations thereof; stabilizers that include antioxidants, preservatives, buffering agents, bases and others known in the art; enzyme inhibitors, p-glycoprotein inhibitors, multidrug resistance protein inhibitors, or any combinations thereof. Preferably, the said enhancer is a solubility enhancer or a dissolution enhancer.

In one embodiment of the present invention, at least part of the EIR topiramate formulation is contained in at least one population of beads.

In a further embodiment, the active ingredient per se is contained in at least one population of beads. Topiramate contained in the beads may be complexed with a cyclodextrin, as described above.

Topiramate containing beads may additionally comprise at least one enhancing agent selected from the group consisting of solubility enhancing agents, dissolution enhancing agents, absorption enhancing agents, penetration enhancing agents, surface active agents, stabilizers, enzyme inhibitors, p-glycoprotein inhibitors, multidrug resistance protein inhibitors and combinations thereof.

In an alternative embodiment, at least one enhancing agent may be contained in at least one population of beads having no active compound. This arrangement is beneficial in cases when the enhancing agent, i.e., a permeability enhancer, does not exhibit the optimal dissolution properties and may negatively impact the dissolution of topiramate. In this embodiment, topiramate or its complex with a complexing agent, may be contained in a separate populations of beads, or it may be in a powder form.

Enhanced immediate release topiramate beads can be prepared using processes suitable for bead manufacturing, such as coating of a topiramate suspension, dispersion or solution onto an inert carrier, or by roller compaction, granulation, extrusion/spheronization, or powder coating, and are not limited by the examples cited herein. Inert carriers useful in the present invention may be selected from, but are not limited to, a group comprising cellulose spheres, silicon dioxide, starch and sugar spheres. The inert carrier is present in an amount of from about 5% to about 99% by weight, and preferably in an amount of from about 20% to about 98% by weight.

By way of a non-limiting example, enhanced immediate release beads containing topiramate were prepared by coating topiramate dispersion onto an inert carrier such as sugar spheres. The topiramate dispersion, in addition to topiramate in micronized form or in non-micronized form, can contain one or more complexing agents or enhancers, water and optionally a binder such as hydroxypropylcellullose, hydroxypropylmethylcellulose, polyvinylpyrrolidone and polyvinyl alcohol.

When a formulation is enhanced with complexing agents, said agents may be first mixed with topiramate and a suitable solvent such as water to form the complex as described above. The topiramate-containing complex is then mixed with a binder solution prepared separately to provide the coating dispersion. The coating dispersion is then sprayed onto the inert carrier such as sugar spheres using a fluid bed processor. Optionally, the beads of the current invention can be additionally coated with an over-coat. The over-coat can be a moisture barrier coat, a protection coat, a seal coat, a taste-masking coat, a flavor coat, a polish coat, a color coat, or any other cosmetic coat that does not interfere with the release of the active compound or the enhancing agent. Suitable coating materials for such an over-coat are known in the art, and include, but are not limited to, cellulosic polymers such as hydroxypropylmethylcellulose, hydroxypropylcellulose and microcrystalline cellulose, or combinations thereof (for example various Opadry® coating materials).

EIR formulations of topiramate of the present invention may be prepared in an oral dosage form, or in any other dosage form represented by the non-limiting examples of aerosols, sprays, injections, suppositories, or patches. The oral dosage form may be selected from a tablet, a pill, a capsule, a caplet, a troche, a sachet, a cachet, a pouch, a powder, a bead, a solution, a gum and sprinkles.

In one embodiment of the invention, the dosage form is an orally disintegrating dosage form (ODDF). These dosage forms, such as fast disintegrating, fast-dissolving or fast-melting tablets or beads, can be taken conveniently in any situation and provide a rapid onset of action desired for some conditions, such as migraine. The incorporation of the enhanced immediate release formulation of the present invention into the orally disintegrating dosage form allows a combined benefit of the rapid onset of action, faster drug dissolution and a better absorption.

The orally disintegrating dosage forms may be prepared by various methods, such as molding, freeze-drying, extrusion, direct compression, spray-drying, and sublimation. The choice of method depends on the desired properties and the size of the final dosage form units. In general, dosage forms comprised of smaller size units achieve faster disintegration and dissolution of the active ingredient due to the enhanced surface area.

The ODDF of the current invention may contain typical excipients well known in the art, including but not limited to highly soluble materials, bulking agents, wetting agents, anti-tack agents, taste-masking agents, buffering agents, disintegrants, lubricants, flow aids, flavoring agents, sweetener, and coloring agents. Highly soluble materials useful in the current invention include, but are not limited to, amino acids such as glutamic acid and glycine, carbohydrates such as mannose, amylose, maltose, mannitol, lactose, sucrose, glucose, xylitose, dextrins, such as maltodextrin; polyethylene glycols, polyalcohols, polyvinyl alcohols, polyvinyl pyrrolidones, and salts such as, sodium bicarbonate, and calcium citrate.

Disintegrants useful in the practice of the current invention include but are not limited to crospovidone, sodium starch glycolate, crosscarmellose sodium, carboxymethylcellulose, starch, pregelatinized starch, substituted hydroxypropylcellulose, microcrystalline cellulose, and compounded disintegrants.

In another embodiment of the invention, the dosage form is a capsule. The enhanced immediate release formulation may be contained in the capsule in the form of a powder, in the form of at least one population of beads, or as a mixture of both. In addition to the EIR formulation of topiramate, the capsule may contain additional pharmaceutical ingredients formulated for immediate or sustained (extended) release. In one embodiment, such additional pharmaceutical ingredient maybe an extended release formulation of topiramate. In the other embodiment, said additional pharmaceutically active agents, without putting any limitation thereon, may be represented by analgesic and anti-inflammatory compounds such as COX-2 inhibitors, nonsteroidal anti-inflammatory drugs (NSAIDs), narcotic drugs such as opiates and morphinomimetics, synthetic drugs with narcotic properties such as tramadol; anticonvulsants such as valproic acid or its derivatives, carbamazepine, oxcarbazepine, gabapentin, and lamotrigine; anorectics or anti-obesity agents such as sibutramine or other, orlistat or other pancreatic lipase inhibitors, diethylpropion, fluoxetine, bupropion, amphetamine, methamphetamine, sertraline, zonisamide, and metformin, as well as medications associated with weight-gain, such as sulfonylurea derivatives, insulin, and thiazolidinediones whose weight-gain effect is tempered by topiramate; antihypertensive agents such as diuretics, anti-adrenergics, calcium channel blockers, ACE inhibitors, angiotensin II receptor antagonists, aldosterone antagonists, vasodilators, centrally acting adrenergic drugs, and adrenergic neuron blockers; mood stabilizers such as various forms/salts of lithium, Omega-3 fatty acids and other known in the art, drugs for treatment or prevention of migraines, such as ergot derivatives or triptans, or any other pharmaceutical or nutraceutical ingredient that can be safely and beneficially combined with topiramate.

In yet another embodiment of the invention, the capsule comprises a first quantity of the EIR topiramate formulation encapsulated inside said capsule, and a second quantity of the EIR topiramate formulation coated as a layer on the outer surface of the capsule. This dosage form can also provide a rapid onset of action in the cases where such onset is desired. This rapid onset however will be followed by a release of the remaining part of the formulation. The EIR coated capsule of the present invention may be prepared by coating capsules with a formulation containing topiramate and a complexing / enhancing agent-containing solution or dispersion in an appropriate coating machine such as a fluid bed or a pan coater. In case of a dispersion, at least part of the undissolved topiramate may be in a micronized form with the particle size from less than 10 µm to less than 100 µm.

The above described "composite" dosage form may be used without any limitations for pharmaceutical ingredients other than topiramate. In general, it consists of a capsule comprising a first pharmaceutical formulation enclosed therein, and a layer of a second pharmaceutical formulation coated on the outer surface of the capsule. In one embodiment, the first pharmaceutical formulation and the second pharmaceutical formulation comprise the same active ingredient. Preferably, the second pharmaceutical formulation is an immediate release formulation providing an immediate onset of action of an active ingredient. The first pharmaceutical formulation contained inside the capsule may be an immediate release formulation, a delayed release formulation, a sustained release formulation, an extended release formulation, or a combination thereof, depending on the desired release parameters and therapeutic indications for the dosage form.

In a further embodiment of the current invention, the EIR topiramate formulation is administered as a powder. The particle size of the powder can be controlled by methods well know in the art, such as spray freeze drying, spray drying, freeze drying, supercritical fluid drying, and so on. The enhanced immediate release topiramate formulation can be made into very fine particles, such as less than 10 microns or less than 5 microns, by an appropriate method, such as spray freeze drying. The fine particles of enhanced immediate release topiramate are suitable for nebulization for delivery to the lung, with enhanced topiramate solubility and bioavailability provided by the complexing agent and/or the enhancers. Said powder may be also reconstituted to form a solution which can be used to deliver doses of topiramate by various administration routes, such as nasally, orally, parenterally or through the pulmonary spray.

It is also an object of the instant invention to present a method of treatment or prevention of a pathological condition in a mammalian subject by administering to said subject an enhanced immediate release topiramate formulation. Pathological conditions that may be treated by a method of the present invention include neurological conditions, psychiatric conditions, diabetes and related disorders, cardiovascular conditions, obesity, and any other condition or disorder that may be treated or prevented by the topiramate administration.

Neurological disorders that may be treated or prevented by a formulation of the present invention include, but are not limited to, epilepsy, migraine, essential tremor, restless limb syndrome, cluster headaches, neuralgia, neuropathic pain, Tourrette's syndrome , infantile spasms, perinatal hypoxia ischemia and related damage, chronic neurodegenerative disorders, acute neurodegeneration, and ALS.

Psychiatric disorders that may be treated or prevented by a formulation of the present invention include, but are not limited to bipolar disorder, dementia, depression, psychosis, mania, anxiety, schizophrenia, obsessive-compulsive disorder, post-traumatic stress disorder, ADHD, impulse control disorders, border line personality disorder, addiction, and autism.

Formulations of the present invention may be also used for the treatment and prevention of diabetes and related disorders, such as type II diabetes mellitus, diabetic retinopathy, impaired oral glucose tolerance, diabetic skin lesions, diabetic neuropathy, Syndrome X and elevated blood glucose levels; ocular disorders, including but not limited to glaucoma and macular degeneration; cardiovascular disorders represented but not limited to elevated blood pressure and elevated lipids; obesity; asthma; autoimmune disorders; sleep apnea and sleep disorders. The formulations may be also used for inducing weight loss or promoting wound healing, or for any other condition, not specified above, wherein the use of topiramate is indicated.

The method of the current invention is advantageously used for a stand-alone or an adjunct treatment of the conditions where a rapid onset of action is essential. These conditions may be represented by pain attacks, acute angle-closure glaucoma, acute neurodegeneration, sleep apnea and sleep disorders, elevated blood glucose levels that involves insulin treatment, a hypertensive emergency, an asthma attack, and a sudden craving associated with an addiction treatment or smoking cessation, among others. In one embodiment of the invention, said condition is a migraine. The enhanced immediate release topiramate formulation may be administered as a stand alone treatment for the acute attacks of migraine as an adjunct treatment for the breakthrough episodes of migraine for subjects receiving the long-term treatment with a migraine preventative medication. Said migraine preventative medication is preferably an extended release formulation of topiramate.

### EXAMPLES

### Example 1. Method of preparation of topiramate complex with hydroxypropyl-beta-cyclodextrin.

Approximately half of the total intended amount of topiramate was added to the water with constant mixing followed by sprinkling of hydroxypropyl-beta-cyclodextrin into the dispersion. Once the dispersion became significantly less viscous, more drug substance was added followed by sprinkling of more hydroxypropyl-beta-cyclodextrin. The drug and hydroxypropyl-beta-cyclodextrin addition steps were repeated, and the dispersion was mixed for 12-18 hours. Separately, a binder such as hydroxypropylmethylcellulose was dissolved in water. The above topiramate - hydroxypropyl-beta-cyclodextrin dispersion and hydroxypropylmethylcellulose solution were mixed together for 15 to 30 minutes and the mixture was screened through an 80-mesh sieve.

### Example 2. Topiramate-Hydroxypropyl-beta-cyclodextrin complex beads

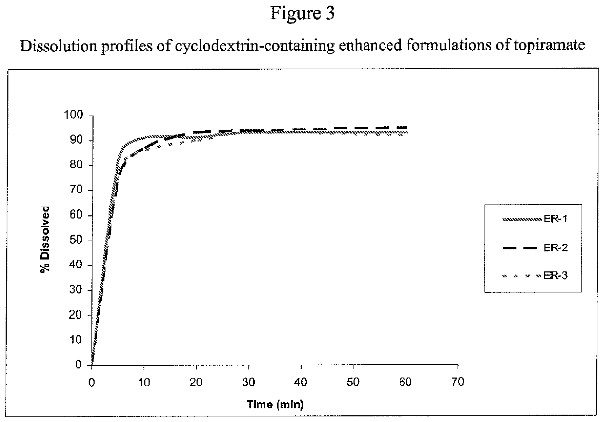

The dispersion of Example 1 was sprayed onto sugar spheres using a fluid bed processor to yield the enhanced immediate release beads. The dissolution profiles of the different enhanced formulation of topiramate can be seen in Figure 3. The composition of the beads is represented in Table 2.

**Table 2. Immediate release topiramate bead compositions enhanced with hydroxypropyl-beta-cyclodextrin**

| Component | Percentage (w/w) in Beads | | | |
|---|---|---|---|---|
| | EIR-1 (HPBCD:Drug = 3:2)* | EIR-2 (HPBCD:Drug = 3:2)* | EIR-3 (HPBCD:Drug = 1:1)* | EIR-4 (HPBCD:Drug = 1:2)* |
| Topiramate | 25.0 | 3.3 | 28.9 | 33.3 |
| Hydroxypropyl-beta-cyclodextrin | 37.5 | 4.95 | 28.9 | 16.7 |
| Hydroxypropylmethylcellu lose | 3.1 | 0.41 | 2.4 | 4.2 |
| Sugar spheres | 34.4 | 91.34 | 39.8 | 45.8 |

| | | | | |
|---|---|---|---|---|
| * HPBCD:Drug - Hydroxypropyl-beta-cyclodextrin to drug substance ratio | | | | |

### Example 3. EIR Topiramate Powders

The solution / dispersion/ suspension of EIR topiramate was prepared with or without the use of a binder or other pharmaceutically acceptable excipients. The process parameters, such as the ratio of topiramate to the complexing and/or enhancing agents, the ratio of topiramate to the media and solvents, and the starting topiramate particle size were controlled in such a way that the undissolved topiramate particles in the dispersion / suspension had a size of from less than 10 µm to less than 100 µm. The EIR topiramate solution / dispersion / suspension was then turned into powder form by an appropriate drying method such as spray drying, freeze drying, spray freeze drying, spraying onto a carrier powder and drying, evaporation, simple drying such as drum drying, dielectric drying such as radiofrequency or microwave drying, or supercritical fluid drying.

### (a) Spray drying

Spray drying can be carried out in a suitable spray dryer, such as a fluidized spray dryer or a multi-stage spray dryer. Specifically, the EIR topiramate solution / dispersion / suspension was sprayed into a fluid bed and dried with heated air. The powder was then collected. Optionally, the powder may be sieved to remove large agglomerates.

### (b) Freeze drying

Freeze drying can be carried out in a suitable freeze dryer. Specifically, the EIR topiramate solution / dispersion/ suspension was frozen and dried under reduced pressure.

### (c) Spray onto a carrier powder

The EIR topiramate solution / dispersion / suspension can be sprayed onto a carrier powder, such as a sugar powder, or other pharmaceutically acceptable excipients powders, and dried. Specifically, the EIR topiramate solution/ dispersion / suspension was sprayed onto a powder such as mannose or a mixture of mannose and sodium lauryl sulfate and optionally other pharmaceutically acceptable excipients, in a fluid bed and dried with heated air. After completing the spraying, the granulation mixture was further dried to remove residue solvents. The granulation powder was then sieved to remove large agglomerates.

Alternatively, the EIR topiramate solution/dispersion/suspension was sprayed onto a carrier powder, such as mannose or a mixture of mannose and sodium lauryl sulfate and optionally other pharmaceutically acceptable excipients, in a granulator. After the spraying, the granules were dried by an appropriate drying method, such as fluid bed drying, or oven drying. The dried granulates were sieved to remove large particles.

### Example 4. EIR topiramate beads containing non-complexing enhancers.

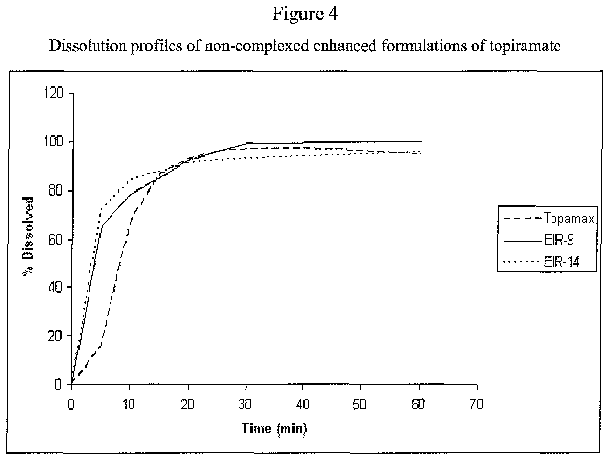

Topiramate was dispersed in a binder solution such as hydroxypropylmethylcellulose solution that contained an appropriate amount of enhancer or enhancers such as d-alpha-tocopheryl polyethylene glycol 1000 succinate (vitamin E TPGS) and sodium lauryl sulfate combination, polyoxyl hydrogenated castor oil (different grades of Cremophor RH), polyglycolized glycerides (different grades of Gelucire), polyglycolized glycerides combined with sodium lauryl sulfate, or combinations thereof. The resultant dispersion was sprayed onto an inert carrier such as sugar spheres using a fluid bed processor to achieve a desired drug load (Table 3). The dissolution profiles of these formulations are presented in Fig. 4.

**Table 3. Enhanced immediate release topiramate bead compositions**

| Component | Percentage (w/w) in Beads | | |
|---|---|---|---|
| | EIR-5 | EIR-6 | EIR-7 |
| Topiramate | 36.8 | 37.9 | 36.4 |
| Sodium lauryl sulfate | 0.7 | 0.5 | - |
| D-alpha-tocopheryl polyethylene glycol 1000 succinate | 7.3 | - | - |
| Polyoxyl hydrogenated castor oil (Cremophor RH40) | - | - | 9.1 |
| Polyglycolized glycerides (Gelucire 50/13) | - | 4.7 | - |
| Hydroxypropylmethyl-cellulose | 4.6 | 4.8 | 4.5 |
| Sugar spheres | 50.6 | 52.1 | 50.0 |

### Example 5. Topiramate EIR Beads Containing Micronized Particles

Micronized or non-micronized topiramate is dispersed in a solution with or without heating, optionally containing dissolution enhancing agents such as mannose, maltose, mannitol, lactose, maltodextrin and sodium starch glucolate, and optionally containing one or more additional enhancers such as PEG3350, sodium lauryl sulfate, sodium docusate, polyoxyethylene sorbitan monooleate and Poloxamers, under such process parameters that topiramate particles that remain undissolved have a particle size of about 1 micron to about 30 micron. A particle size reduction device such as a homogenizer can also be used to reduce the particle size of undissolved topiramate (Table 4).

**Table 4. Topiramate Particle size**

| | EIR-9 | EIR-14 |
|---|---|---|
| Undissolved Topiramate particle size (D90) in dispersion | 3 micron | 6 micron |

The resultant topiramate dispersion is then sprayed onto inert carriers such as sugar spheres in a coating processor such as a fluid bed processor. The formulations obtained are represented in the Table 5:

**Table 5. Topiramate EIR Beads containing micronized particles**

| | Percentage (w/w) in Beads | | | | | | |
|---|---|---|---|---|---|---|---|
| | EIR-8 | EIR-9 | EIR-10 | EIR-11 | EIR-12 | EIR-13 | EIR-14 |
| Topiramate | 3.2 | 26.0 | 25.0 | 3.2 | 26.0 | 26.0 | 26.0 |
| Mannose | 0.4 | 5.0 | 3.3 | 2.0 | 10.0 | 10.0 | 10.0 |
| Maltrin 250 | - | - | 1.0 | 1.0 | - | - | - |
| PEG3350 | 1.0 | 15.0 | - | - | - | 10.0 | - |
| Sodium lauryl sulfate | - | - | - | - | 0.5 | - | - |
| Sodium docusate | - | - | - | - | - | - | 0.5 |
| Hydroxypropyl-beta-cyclodextrin | - | - | 37.5 | - | - | - | - |
| D-alpha-tocopheryl Polyethylene glycol 1000 succinate | - | - | - | - | 2.00 | - | - |
| Polyoxyl hydrogenated castor oil (Cremophor RH40) | - | - | - | - | - | 2.0 | - |
| Sugar spheres | 95.4 | 54.0 | 33.2 | 93.8 | 61.5 | 52.0 | 63.5 |

### Example 6. Orally disintegrating enhanced immediate release topiramate tablets and pellets

EIR topiramate powders or granules, as described in previous examples, are blended with additional excipients such as highly soluble materials, disintegrants, fillers, binders, wetting agents, lubricants, anti-tack agents, taste masking agents, flavorants, colorants, buffering agents, and additional enhancers, and made into tablets using a tablet press, or into pellets using a roller compactor. Some of the excipients, such as highly soluble excipients, can be pre-granulated with another excipient before being mixed with the rest of the components in the composition. The compositions of the resulting tablet can be found in Table 6.

**Table 6. Orally disintegrating enhanced immediate release topiramate tablet compositions**

| Component | Percentage (w/w) in Tablets | | |
|---|---|---|---|
| | EIR-15 | EIR-16 | EIR-17 |
| Topiramate | 12.0* | 10.8* | 12.0** |
| Hydroxypropyl-beta-cyclodextrin | 24.0* | 16.3* | - |
| PEG3350 | - | - | 7.0** |
| Mannose | 55.0 | - | 74.5 |
| Mannitol | 5.8 | - | 5.8** |
| Lactose/Starch (Star Lac) | - | 69.5 | - |
| Crosscarmellose sodium | 2.5 | 2.1 | - |
| Silicon dioxide (AEROSIL) | - | 0.5 | - |
| Sodium lauryl sulphate | - | 0.3 | - |
| Magnesium stearate | 0.7 | 0.5 | 0.7 |

| | | | |
|---|---|---|---|
| *As pre-formed complex ** As pre-formed granules or sprayed-dried mixture | | | |

### Example 7. Rapid onset topiramate capsules

Enhanced immediate release topiramate formulation is applied to the outer surface of a capsule, which may further contain an immediate release or an extended release formulation of topiramate, or a combination of such formulations of topiramate. Specifically, topiramate containing powder or beads are filled in a capsule. Optionally, the capsule is coated with a release controlling coating such as methacrylic polymers (Eudragit L30D-55 or Eudragit FS 30 D), an overcoat such as cellulosic polymers (Opadry), or both. An appropriate amount of topiramate complex solution or dispersion or suspension, for example, equivalent to 25 mg of topiramate, is then coated onto the surface of the resultant capsule.

According to a preferred embodiment of the formulation of claim 18, the topiramate is complexed with a cyclodextrin.

According to another preferred embodiment of the formulation of claim 20, said enhancing agent is selected from a group consisting of Vitamin E TPGS, glutamic acid, glycine, sorbitol, mannose, amylose, maltose, mannitol, lactose, sucrose, glucose, xylitose, dextrins, glycerol-polyethylene glycol oxystearate, PEG-32 glyceryl palmitostearate, sodium lauryl sulfate, polyoxyethylene sorbitan monooleate, benzyl alcohol, sorbitan monolaurate, Poloxamer 407, polyethylene glycols, polyvinylpyrrolidones, polyalcohols, polyvinyl alcohols, oleic acid, glyceryl monooleate, sodium benzoate, cetyl alcohol, sucrose stearate, crospovidone, sodium starch glycolate, croscarmellose sodium, carboxymethylcellulose, starch, pregelatinized starch, HPMC, substituted hydroxypropylcellulose, microcrystalline cellulose sodium bicarbonate, calcium citrate, sodium docusate, and menthol.

A preferred embodiment is directed to a method of treatment or prevention of a pathological condition in a mammalian subject, comprising administering to said subject a therapeutically effective amount of an enhanced immediate release (EIR) topiramate formulation comprising topiramate and at least one agent selected from a group consisting of complexing agents, enhancing agents and combinations thereof, at least 80 % of topiramate is dissolved in a time period of not more than 30 minutes:
wherein preferably at least 30% of the active compound is dissolved in not more than 5 min;
wherein preferably said complexing agent is selected from a group consisting of cyclodextrins, benzoates, hydroxybenzoates, polyvinylpyrrolidones, pyridoxine HCl, nicotinamide, polyamines, polyamides, polyethyleneimines, polyvinylpyridine, polylysine, aminopolysaccharides, chitosan, polyanions, oxa- and thia- crown ethers, polyoxalkylenes, and polysiloxanes;
wherein preferably said complexing agent is a cyclodextrin selected from a group consisting of hydroxypropyl-beta-cyclodextrin, beta-cyclodextrin, gamma-cyclodextrin, and alpha-cyclodextrin, or a cyclodextrin derivative;
wherein preferably said enhancing agent is selected from a group consisting of solubility enhancing agents, dissolution enhancing agents, absorption enhancing agents, penetration enhancing agents, surface active agents, stabilizing agents, enzyme inhibitors, p-glycoprotein inhibitors, multidrug resistance protein inhibitors and combinations thereof;
wherein preferably said enhancing agent is selected from a group consisting of Vitamin E TPGS, glutamic acid, glycine, sorbitol, mannose, amylose, maltose, mannitol, lactose, sucrose, glucose, xylitose, dextrins, glycerol-polyethylene glycol oxystearate, PEG-32 glyceryl palmitostearate, sodium lauryl sulfate, polyoxyethylene sorbitan monaoleafie, benzyl alcohol, sorbitan monolaurate, Poloxamer, polyethylene glycols, polyvinylpyrrolidones, polyalcohols, polyvinyl alcohols, oleic acid, glyceryl monooleate, sodium benzoate, cetyl alcohol, sucrose stearate, crospovidone, sodium starch glycolate, croscaramellose sodium, carboxymethylcellulose,
starch, pregelatinized starch, HPMC, substituted hydroxypropylcellulose, microcrystalline cellulose sodium bicarbonate, calcium citrate, sodium docusate, and menthol;
wherein preferably said formulation is administered orally in a dosage form selected from a tablet, a pill, a capsule, a caplet, a bead, a troche, a sachet, a cachet, a pouch, a powder, a solution, a gum, sprinkles, and an orally disintegrating dosage form;
wherein preferably said formulation is administered as the orally disintegrating dosage form;
wherein preferably said formulation is administered in a total dose of from 0.5 to 3000 mg;
wherein preferably at least part of said active ingredient is in a form of micronized particles;
wherein preferably said particles have an average size of from about 1 µm to about 100 µm.

According to a preferred embodiment of the invention, the pharmaceutical dosage form comprises a therapeutically effective amount of an enhanced immediate release topiramate formulation comprising topiramate and at least one agent selected from a group consisting of complexing agents, enhancing agents and combinations thereof, wherein at least 80 % of topiramate is dissolved in a time period of not more than 30 minutes:
wherein preferably at least 30% of the active compound is released in the time period of not more that 5 minutes;
wherein preferably said complexing agent is a cyclodextrin selected from a group comprising hydroxypropyl-beta-cyclodextrin, beta-cyclodextrin, gamma-cyclodextrin, and alpha-cyclodextrin, or a cyclodextrin derivative;
wherein preferably said enhancing agent is selected from a group comprising solubility enhancing agents, dissolution enhancing agents, absorption enhancing agents, penetration enhancing agents, surface active agents, stabilizing agents, enzyme inhibitors, p-glycoprotein inhibitors, multidrug resistance protein inhibitors and combinations thereof;
wherein preferably said formulation is administered in a dosage form selected from an aerosol, a spray, an injection, a suppository, and a patch;
wherein preferably said formulation is administered orally;
wherein preferably the dosage form is selected from a tablet, a pill, a capsule, a caplet, a bead, a troche, a sachet, a cachet, a pouch, a powder, a solution, a gum, sprinkles and an orally disintegrating dosage form;
wherein preferably said orally disintegrating dosage form is a fast-dissolving tablet or a fast-disintegrating tablet;
wherein preferably said tablet is prepared by a process comprising the steps of:
   1. preparing a solution or dispersion of topiramate with at least one of the complexing agents, enhancing agents, or a combination thereof;
   2. drying the result of step 1;
   3. adding at least one agent selected from bulking agents, disintegrating agents, binders, lubricants, flow aids, wetting agents, anti-tack agents, buffering agents flavoring agents, sweetener, coloring agents, highly soluble materials and combinations thereof to the result of step 2;
   4. forming the result of step 3 into a tablet;
wherein preferably said formulation comprises a complex with topiramate with hydroxypropyl-beta-cyclodextrin;
wherein preferably said formulation additionally comprises at least one enhancing agent selected from a group consisting of solubility enhancing agents, dissolution enhancing agents, absorption enhancing agents, penetration enhancing agents, surface active agents, stabilizing agents, enzyme inhibitors, p-glycoprotein inhibitors, multidrug resistance protein inhibitors and combinations thereof;
wherein preferably the dosage form is a capsule;
wherein preferably at least part of the EIR topiramate formulation is contained in said capsule in the form of at least one population of beads;
wherein preferably at least part of said active ingredient is in a form of micronized particles;
wherein preferably said particles have an average size of from about 1 µm to about 100 µm.

According to a preferred embodiment of a pharmaceutical dosage form, the dosage form comprises a capsule containing a first pharmaceutical formulation enclosed therein, and a layer of a second pharmaceutical formulation coated on an outer surface of the capsule:
wherein preferably the first pharmaceutical formulation and the second pharmaceutical formulation comprise the same active ingredient;
wherein preferably the second pharmaceutical formulation is an immediate release formulation;
wherein preferably the first pharmaceutical formulation is an immediate release formulation, a delayed release formulation, a sustained release formulation, an extended release formulation, or a combination thereof;
wherein preferably at least the second pharmaceutical formulation comprises topiramate as an active ingredient;
wherein preferably at least part of said active ingredient is in a form of micronized particles;
wherein preferably said particles have an average size of from about 1 µm to about 100 µm.

## Claims

1. An enhanced immediate release topiramate formulation for administration to a mammalian subject comprising topiramate and at least one agent selected from a group consisting of complexing agents, enhancing agents and combinations thereof, wherein at least 80 % of topiramate is dissolved in a time period of not more than 60 minutes, and wherein said formulation comprises at least one population of beads that contains at least one enhancing agent and contains no topiramate.

2. The formulation of claim 1 wherein said complexing agent is selected from a group consisting of cyclodextrins, benzoates, hydroxybenzoates, polyamides, polyvinylpyrrolidones, pyridoxine HCl, nicotinamide, polyamines, polyethyleneimines, polyvinylpyridine, polylysine, aminopolysaccharides, chitosan, polyanions, oxa- and thia- crown ethers, polyoxalkylenes, and polysiloxanes.

3. The formulation of claim 2 wherein said complexing agent is the cyclodextrin selected from a group consisting of hydroxypropyl-beta-cyclodextrin, beta-cyclodextrin, gamma-cyclodextrin, and alpha-cyclodextrin, or its derivative.

4. The formulation of claim 1 wherein said enhancing agent is selected from a group consisting of solubility enhancing agents, dissolution enhancing agents, absorption enhancing agents, penetration enhancing agents, surface active agents, stabilizing agents, enzyme inhibitors, p-glycoprotein inhibitors, multidrug resistance protein inhibitors and combinations thereof.

5. The formulation of claim 4, wherein said enhancing agent is selected from a group consisting of Vitamin E TPGS, glutamic acid, glycine, sorbitol, mannose, amylose, maltose, mannitol, lactose, sucrose, glucose, xylitose, dextrins, glycerol-polyethylene glycol oxystearate, PEG-32 glyceryl palmitostearate, sodium lauryl sulfate, polyoxyethylene sorbitan monooleate, benzyl alcohol, sorbitan monolaurate, Poloxamer 407, polyethylene glycols, polyvinylpyrrolidones, polyalcohols, polyvinyl alcohols, oleic acid, glyceryl monooleate, sodium benzoate, cetyl alcohol, sucrose stearate, crospovidone, sodium starch glycolate, croscarmellose sodium, carboxymethylcellulose, starch, pregelatinized starch, HPMC, substituted hydroxypropylcellulose, microcrystalline cellulose sodium bicarbonate, calcium citrate, sodium docusate, and menthol.

6. The formulation of claim 3 comprising a complex of topiramate and hydroxypropyl-beta-cyclodextrin.

7. The formulation of claim 1, wherein at least part of said active ingredient is in a form of micronized particles.

8. The formulation of claim 7, wherein said particles have an average size of from about 1 µm to about 100 µm.

9. The formulation of claim 1 wherein the amount of topiramate in the formulation is from 0.5 to 3000 mg.

10. The formulation of claim 1, wherein said formulation is administered in a dosage form selected from an aerosol, a spray, an injection, a suppository, and a patch.

11. The formulation of claim 1 wherein said formulation is administered orally.

12. The formulation of claim 11, wherein said formulation is in a dosage form selected from a tablet, a pill, a capsule, a caplet, a bead, a troche, a sachet, a cachet, a pouch, a powder, a solution, a gum, sprinkles and an orally disintegrating dosage form.

13. The formulation of claim 1, wherein topiramate is contained in at least one population of beads.

14. The formulation of any of the claims 1-14 for use in a method of treatment or prevention of a pathological condition in a mammalian subject wherein said condition is selected from a group consisting of epilepsy, migraine, essential tremor, restless limb syndrome, cluster headaches, neuralgia, neuropathic pain, Tourrette's syndrome, infantile spasms, perinatal hypoxia ischemia and related damage, glaucoma, ocular disorders, obesity, weight loss, Type II diabetes mellitus, diabetic retinopathy, impaired oral glucose tolerance, diabetic skin lesions, diabetic neuropathy, elevated blood glucose levels, syndrome X, elevated blood pressure, elevated lipids, bipolar disorder, dementia, depression, psychosis, mania, anxiety, schizophrenia, obsessive-compulsive disorder, post-traumatic stress disorder, ADHD, impulse control disorders, border line personality disorder, addiction, autism, asthma, autoimmune disorders, chronic neurodegenerative disorders, acute neurodegeneration, ALS, sleep apnea and sleep disorders.

## Patentansprüche

1. Topiramat-Zubereitung mit verstärkter sofortiger Freisetzung zur Verabreichung an einen Säuger, umfassend Topiramat und mindestens ein Mittel, das aus einer Gruppe ausgewählt ist, die besteht aus Komplexierungsmitteln, Verstärkungsmitteln und Kombinationen davon, wobei mindestens 80 % des Topiramats innerhalb einer Zeitspanne von nicht mehr als 60 Minuten in Lösung gehen und wobei die Zubereitung mindestens eine Population von Kügelchen umfasst, die mindestens ein Verstärkungsmittel enthält und kein Topiramat enthält.

2. Zubereitung nach Anspruch 1, wobei das Komplexierungsmittel aus einer Gruppe ausgewählt ist, die besteht aus Cyclodextrinen, Benzoaten, Hydroxybenzoaten, Polyamiden, Polyvinylpyrrolidonen, Pyridoxin-HCl, Nicotinamid, Polyaminen, Polyethyleniminen, Polyvinylpyridin, Polylysin, Aminopolysacchariden, Chitosan, Polyanionen, Oxa- und Thia-kroneethern, Polyoxalkylenen und Polysiloxanen.

3. Zubereitung nach Anspruch 2, wobei es sich beim Komplexierungsmittel um Cyclodextrin, das aus einer Gruppe ausgewählt ist, die besteht aus Hydroxypropyl-beta-cyclodextrin, beta-Cyclodextrin, gamma-Cyclodextrin und alpha-Cyclodextrin, oder ein Derivat davon handelt.

4. Zubereitung nach Anspruch 1, wobei das Verstärkungsmittel aus einer Gruppe ausgewählt ist, die besteht aus Löslichkeitsverstärkungsmitteln, Auflösungsverstärkungsmitteln, Absorptionsverstärkungsmitteln, Penetrationsverstärkungsmitteln, oberflächenaktiven Mitteln, Stabilisierungsmitteln, Enzym-Inhibitoren, p-Glycoprotein-Inhibitoren, Multidrug-Resistance-Protein-Inhibitoren und Kombinationen davon.

5. Zubereitung nach Anspruch 4, wobei das Verstärkungsmittel aus einer Gruppe ausgewählt ist, die besteht aus Vitamin E-TPGS, Glutaminsäure, Glycin, Sorbitol, Mannose, Amylose, Maltose, Mannitol, Lactose, Saccharose, Glucose, Xylitose, Dextrinen, Glycerinpolyethylenglycol-oxystearat, PEG-32-Glyceryl-palmitostearat, Natriumlaurylsulfat, Polyoxyethlensorbitan-monooleat, Benzylalkohol, Sorbitan-monolaurat, Poloxamer 407, Polyethylenglcolen, Polyvinylpyrrolidonen, Polyalkoholen, Polyvinylalkoholen, Ölsäure, Glycerylmonooleat, Natriumbenzoat, Cetylalkohol, Saccharosestearat, Crospovidon, Natriumstärkeglycolat, Croscarmellose-Natrium, Carboxymethylcellulose, Stärke, vorgelierte Stärke, HPMC, substituierter Hydroxypropylcellulose, mikrokirstalliner Cellulose, Natriumbicarbonat, Calciumcitrat, Natriumdocusat und Menthol.

6. Zubereitung nach Anspruch 3, umfasssend einen Komplex aus Topiramat und Hydroxypropyl-beta-cyclodextrin.

7. Zubereitung nach Anspruch 1, wobei mindestens ein Teil des Wirkstoffs in Form von mikronisierten Teilchen vorliegt.

8. Zubereitung nach Anspruch 7, wobei die Teilchen eine durchschnittliche Größe von etwa 1 µm bis etwa 100 µm aufweisen.

9. Zubereitung nach Anspruch 1, wobei die Menge an Topiramat in der Zubereitung 0,5 bis 3000 mg beträgt.

10. Zubereitung nach Anspruch 1, wobei die Zubereitung in einer Dosierungsform verbreicht wird, die ausgewählt ist aus einem Aerosol, einem Sprühmittel, einem Injektionspräparat, einem Suppositorium und einem Pflaster.

11. Zubereitung nach Anspruch 1, wobei die Zubereitung oral verabreicht wird.

12. Zubereitung nach Anspruch 11, wobei die Zubereitung in einer Dosierungsform verabreicht wird, die ausgewählt ist aus einer Tablette, einer Pille, einer Kapsel, einem Caplet, einem Kügelchen, einer Pastille, einem Sachet, einem Cachet, einem Tütchen, einem Pulver, einer Lösung, einer Gumme, Sprühmitteln und einer Lingualtabeltte.

13. Zubereitung nach Anspruch 1, wobei Topiramat in mindestens einer Population von Kügelchen enthalten ist.

14. Zubereitung nach einem der Ansprüche 1-14, zur Verwendung in einem Verfahren zur Therapie oder Prophylaxe eines pathologischen Zustands bei einem Säuger, wobei der Zustand aus einer Gruppe ausgewählt ist, die besteht aus Epilepsie, Migräne, essentiellem Tremor, Restless-Limb-Syndrom, Cluster-Kopfschmerzen, Neuralgie, neuropathischen Schmerzen, Tourette-Syndrom, infantilen Spasmen, perinataler Hypoxie-Ischämie und verwandten Schädigungen, Glaukom, Augenstörungen, Fettleibigkeit, Gewichtsverlust, Diabetes mellitus Typ II, diabetischer Retinopathie, gestörter oraler Glucose-Toleranz, diabetischen Hautläsionen, diabetischer Neuropathie, erhöhtem Blutzuckerspiegel, Syndrom X, erhöhtem Blutdruck, erhöhten Lipiden, bipolarer Störung, Demenz, Depression, Psychose, Manie, Angstzuständen, Schizophrenie, Zwangsstörung, posttraumatischer Stress-Störung, ADHD, Impulskontrollstörung, Borderline-Persönlichkeitsstörung, Suchtstörungen, Autismus, Asthma, Autoimmunstörungen, chronischen neurodegernativen Störungen, akuter Neurodegeneration, ALS, Schlafapnoe und Schlafstörungen.

## Revendications

1. Formulation à libération immédiate de topiramate améliorée pour administration à un sujet mammifère comprenant du topiramate et au moins un agent choisi dans un groupe constitué des agents complexants, des agents améliorants et des combinaisons de ceux-ci, dans laquelle au moins 80 % de topiramate sont dissous en un laps de temps non supérieur à 60 minutes, et dans laquelle ladite formulation comprend au moins une population de perles qui contient au moins un agent activant et qui ne contient pas de topiramate.

2. Formulation selon la revendication 1 dans laquelle ledit agent complexant est choisi dans un groupe constitué des cyclodextrines, des benzoates, des hydroxybenzoates, des polyamides, des polyvinylpyrrolidones, de la pyridoxine HCl, du nicotinamide, des polyamines, des polyéthylèneimines, de la polyvinylpyridine, de la polylysine, des aminopolysaccharides, du chitosane, des polyanions, des éthers oxa- et thia-couronne, des polyoxalkylènes et des polysiloxanes.

3. Formulation selon la revendication 2 dans laquelle ledit agent complexant est la cyclodextrine choisie dans un groupe constitué de l'hydroxypropyl-bêta-cyclodextrine, de la bêta-cyclodextrine, de la gamma-cyclodextrine et de l'alpha-cyclodextrine, ou de ses dérivés.

4. Formulation selon la revendication 1 dans laquelle ledit agent activant est choisi dans un groupe constitué des agents améliorant la solubilité, des agents améliorant la dissolution, des agents améliorant l'absorption, des agents améliorant la pénétration, des tensioactifs, des agents de stabilisation, des inhibiteurs d'enzyme, des inhibiteurs de p-glycoprotéine, des inhibiteurs de protéine multirésistante et des combinaisons de ceux-ci.

5. Formulation selon la revendication 4, dans laquelle ledit agent activant est choisi dans un groupe constitué de la vitamine E TPGS, de l'acide glutamique, de la glycine, du sorbitol, du mannose, de l'amylose, du maltose, du mannitol, du lactose, du sucrose, du glucose, du xylitose, des dextrines, de l'oxystéarate de glycérol-polyéthylène glycol, du palmitostéarate de PEG-32 glycéryle, du laurylsulfate de sodium, du monooléate de polyoxyéthylène sorbitan, de l'alcool benzylique, du monolaurate de sorbitan, du Poloxamer 407, des polyéthylène glycols, des polyvinylpyrrolidones, des polyalcools, des alcools polyvinyliques, de l'acide oléique, du monooléate de glycéryle, du benzoate de sodium, de l'alcool cétylique, du stéarate de sucrose, de la crospovidone, du glycolate d'amidon sodique, de la croscarmellose sodique, de la carboxyméthylcellulose, de l'amidon, de l'amidon prégélatinisé, de l'HPMC, de l'hydroxypropylcellulose substituée, du bicarbonate de cellulose sodique microcristalline, du citrate de calcium, du docusate de sodium et du menthol.

6. Formulation selon la revendication 3 comprenant un complexe de topiramate et d'hydroxypropyl-béta-cyclodextrine.

7. Formulation selon la revendication 1, dans laquelle au moins une partie dudit principe actif se présente sous la forme de particules micronisées.

8. Formulation selon la revendication 7, dans laquelle lesdites particules ont une taille moyenne d'environ 1 µm à environ 100 µm.

9. Formulation selon la revendication 1 dans laquelle la quantité de topiramate dans la formulation est de 0,5 à 3 000 mg.

10. Formulation selon la revendication 1, dans laquelle ladite formulation est administrée sous une forme galénique choisie parmi un aérosol, un spray, une injection, un suppositoire et un timbre.

11. Formulation selon la revendication 1 dans laquelle ladite formulation est administrée par voie orale.

12. Formulation selon la revendication 11, dans laquelle ladite formulation se présente sous une forme galénique choisie parmi un comprimé, une pilule, une capsule, un comprimé-capsule, une perle, un trochisque, un sachet, un cachet, un sac, une poudre, une solution, une gomme, des comprimés à saupoudrer et sous une forme galénique se délitant dans la bouche.

13. Formulation selon la revendication 1, dans laquelle le topiramate est renfermé par au moins une population de perles.

14. Formulation selon l'une quelconque des revendications 1 à 14 pour utilisation dans un procédé de traitement ou de prévention d'un état pathologique chez un sujet mammifère, dans laquelle ledit état est choisi dans un groupe constitué de l'épilepsie, de la migraine, du tremblement essentiel, du syndrome des membres sans repos, des algies vasculaires de la face, de la névralgie, de la douleur neuropathique, du syndrome de Tourrette, des spasmes infantiles, de l'hypoxie-ischémie périnatale et des dommages associés, du glaucome, des troubles oculaires, de l'obésité, de la perte de poids, du diabète de type 2, de la rétinopathie diabétique, de la diminution de la tolérance au glucose oral, des lésions cutanées d'origine diabétique, de la neuropathie diabétique, des taux de glycémie élevés, du syndrome X, de la pression artérielle élevée, des taux de lipides élevés, du trouble bipolaire, de la démence, de la dépression, de la psychose, de la manie, de l'anxiété, de la schizophrénie, du trouble obsessionnel-compulsif, du syndrome de stress post-traumatique, du trouble déficitaire de l'attention avec hyperactivité, des troubles du contrôle des impulsions, du trouble de la personnalité limite, de l'addiction, de l'autisme, de l'asthme, des troubles auto-immuns, des troubles neurodégénératifs chroniques, de la neurodégénérescence aiguë, de la sclérose latérale amyotrophique, de l'apnée du sommeil et des troubles du sommeil.
